(19) Europäisches Patentamt

European Patent Office

Office européen des brevets

(11) **EP 1 700 604 A1**

(12) **EUROPEAN PATENT APPLICATION**
published in accordance with Art. 158(3) EPC

(43) Date of publication:
**13.09.2006 Bulletin 2006/37**

(51) Int Cl.:
*A61K 36/03* (2006.01)    *A23L 1/30* (2006.01)
*A61P 3/10* (2006.01)    *A61P 43/00* (2006.01)
*C12N 9/99* (2006.01)

(21) Application number: **04820260.0**

(22) Date of filing: **09.12.2004**

(86) International application number:
**PCT/JP2004/018370**

(87) International publication number:
**WO 2005/056035 (23.06.2005 Gazette 2005/25)**

(84) Designated Contracting States:
**AT BE BG CH CY CZ DE DK EE ES FI FR GB GR HU IE IS IT LI LT LU MC NL PL PT RO SE SI SK TR**

(30) Priority: **10.12.2003 JP 2003412196**

(71) Applicant: **RIKEN VITAMIN CO., LTD.**
**Chiyoda-ku,**
**Tokyo 101-8370 (JP)**

(72) Inventors:
• **FUNAYAMA, Katsura**
  **Asaka-shi,**
  **Saitama 3510011 (JP)**
• **KAHARA, Takashi**
  **Tokyo 165-0025 (JP)**

• **TANAKA, Minoru**
  **Itabashi-ku,**
  **Tokyo 1740072 (JP)**
• **IIZUKA, Mariko**
  **Saitama 344- 0117 (JP)**
• **IKEDA, Katsumi**
  **Ashiya-shi,**
  **Hyogo 6590064 (JP)**
• **YAMAMOTO, Junko**
  **Kyoto-shi,**
  **Kyoto 6060832 (JP)**

(74) Representative: **Keller, Günter et al**
**Lederer & Keller**
**Patentanwälte**
**Prinzregentenstrasse 16**
**80538 München (DE)**

(54) **ALGA EXTRACT AND SUGAR HYDROLASE INHIBITOR CONTAINING THE SAME**

(57)    A glycosidase inhibitor comprising as an active ingredient an extract of Ascophyllum nodsum which is a kind of brown algae can be used as a useful healthy food or food for specified health uses for the treatment and/or prevention of diabetes.

**Description**

TECHNICAL FIELD

The present invention relates to a glycosidase inhibitor containing as an active ingredient a marine algae extract, more particularly, an extract of Ascophyllum nodsum which is a kind of brown algae.

BACKGROUND ART

Diseases such as obesity, diabetes and the like have kept increasing due to excess calorie intake as a main cause. Diabetes includes two types, one is insulin dependent diabetes (type 1 diabetes) and the other is insulin non-dependent diabetes (type 2 diabetes), and 90% of the total diabetics are patients of the latter type.
Correction of postprandial hyperglycemia in insulin non-dependent diabetes is sometimes difficult even if an oral hypoglycemic agent or insulin is used. Therefore, as means for preventing rapid absorption of carbohydrates in the digestive tract, substances inhibiting the activity of an enzyme correlated with the digestion of ingested carbohydrates are used.
It has been clarified that a substance of inhibiting glycosidase specifically can inhibit glycosidase, as a result, hydrolysis and absorption of carbohydrates are delayed, and rapid increase in the blood glucose level after meal and subsequent insulin increase are suppressed. As such a glycosidase inhibitor, acarbose (trade name: GLUCOBAY; Bayer Yakuhin Ltd.) inhibiting $\alpha$-amylase and $\alpha$-glucosidase and voglibose (trade name:BASEN; Takeda Chemical Industries, Ltd.) inhibiting $\alpha$-glucosidase are actually used clinically as a medicine. However, these medicines need strict prescription by a physician, and it is needless to say that these medicines cannot be utilized in foods.
A glycosidase inhibiting substance or a food containing a glycosidase inhibiting substance added is useful for a patient suffering from dysbolism correlated with glycosidase since pathological conditions of the above-mentioned diseases can be improved, and further, such a glycosidase inhibiting substance or a food containing the same is also suitable for prevention of diabetes by incorporating it into daily eating habits. Therefore, as a highly safe and edible natural substance, there have hitherto been suggested glycosidase inhibiting substances derived from marine algae (see, e.g., patent literatures 1, 2 and 3: JP-A-5-284937, JP-A-2000-342224 and JP-A-2002-212095), wheat flour (see, e.g., patent literature 4: JP-A-57-140727), guava leaf (see, e.g., patent literature 5: JP-A-7-59539), clove (see, e.g., patent literature 6: JP-A-12-072682), edible mushroom (see, e.g., patent literature 7: JP-A-2000-063281), tamarind seed coat (see, e.g., patent literature 8: JP-A-9-291039) and the like.
However, conventional glycosidase inhibiting substances derived from natural substances have weak activity, not reaching sufficiently satisfactory level.
On the other hand, Ascophyllum nodsum is a marine algae belonging to brown algae, Fucales, Fucaceae, and mainly inhabits on the shore reef of ria shoreline in Norway. Ascophyllum nodsum is used as a raw material for producing alginic acid, because it contains alginic acid in high concentration. In addition, since Ascophyllum nodsum contains abundantly minerals and vitamins, a product obtained by drying of a raw alga and pulverization thereof is widely used as a feedstuff or a fertilizer and/or a soil improving agent. However, an extract of Ascophyllum nodsum has not been known to have an inhibitory action on glycosidase.

DISCLOSURE OF THE INVENTION

It is an object of the present invention to provide a glycosidase inhibitor having stronger activity derived from natural substances.
The present inventors have intensively studied to solve the above-mentioned problem, and as a result, have found that an extract of Ascophyllum nodsum which is a kind of brown algae has a strong $\alpha$-amylase inhibitory action, and thus completed the present invention based on this finding.
That is, the present invention relates to:

　　(1) a glycosidase inhibitor comprising an extract of Ascophyllum nodsum as an active ingredient,
　　(2) a glycosidase inhibitor comprising a purified substance of the extract according to the above (1) as an active ingredient,
　　(3) the glycosidase inhibitor according to the above (1) or (2), which is in the form of food and drink,
　　(4) the glycosidase inhibitor according to the above (3), which is in the form of healthy food or food for specified health uses for the treatment and/or prevention of obesity,
　　(5) a method of inhibiting glycosidase, which comprises administering an extract of Ascophyllum nodsum to a mammal,
　　(6) a method of treating or preventing diabetes, which comprises administering an extract of Ascophyllum nodsum to a mammal,

(7) use of an extract of Ascophyllum nodsum for producing a medicine or food and drink which inhibits glycosidase, and

(8) use of an extract of Ascophyllum nodsum for producing a medicine or food and drink for treating or preventing diabetes.

EFFECT OF THE INVENTION

The extract from Ascophyllum nodsum used in the present invention has a strong $\alpha$-amylase inhibitory action and further, also an $\alpha$-glucosidase inhibitory action. Thus, a glycosidase inhibitor comprising the above-mentioned extract can treat and/or prevent diabetes more effectively as compared with conventionally known glycosidase inhibiting substances derived from marine algae.

The glycosidase inhibitor of the present invention is useful for patients suffering from dysbolism (for example, diabetes and the like) correlated with glycosidase, and can be incorporated in food and drink, particularly, in healthy food or food for specified health uses for daily eating habits.

BEST MODES FOR CARRYING OUT THE INVENTION

In the present invention, any tissues and portions of Ascophyllum nodsum (hereinafter, abbreviated as Ascophyllum), preferably leaf and stem parts of algae can be used. In extracting from Ascophyllum, total algae or leaf and stem parts of Ascophyllum harvested from the sea can be used as they are, or they can be cut, finely cut or ground, or dried them. Furthermore, total algae or leaf and stem parts of algae which is cut, finely cut or grounded after drying can be used. Preferably, the whole algae or leaf and stem parts of raw Ascophyllum which is grounded after drying can be used. Drying may be carried out by any methods known per se, for example, air drying, sun drying, freeze drying and the like.

As the extraction solvent, water or organic solvents, or mixed solutions thereof are used. Examples of the organic solvent include polar organic solvents such as lower alcohols having 1 to 4 carbon atoms such as methanol, ethanol, propanol, isopropanol, n-butanol, isobutanol, sec-butanol, tert-butanol and the like, and ketones such as dimethyl ketone, methyl ethyl ketone, acetone, methyl isobutyl ketone and the like; and non-polar organic solvents such as methyl acetate, ethyl acetate, butyl acetate, diethyl ether and the like. These polar organic solvents and non-polar organic solvents can also be used in appropriate combination.

Of these extraction solvents, preferable are polar organic solvents or mixed solutions of polar organic solvents and water, more preferable are methanol, ethanol or acetone or mixed solutions of them and water, and particularly preferable are mixed solutions of methanol, ethanol or acetone and water. The mixing ratio of a polar organic solvent to water varies depending on the kind of a polar organic solvent, and usually, polar organic solvent/water is in a range from about 5/95 to 100/0 (v/v). When a methanol-water mixed solution or ethanol-water mixed solution is used as the extraction solvent, the ratio is about 5/95 to 100/0 (v/v), preferably about 30/70 to 70/30 (v/v). When an acetone-water mixed solution is used, the ratio is about 5/95 to 100/0 (v/v), preferably about 30/70 to 80/20 (v/v). These ratios are preferably determined taking extraction efficiency, amount of extracted substance, enzyme inhibitory activity of extracts, and the like into consideration.

In the present invention, the extraction method for obtaining an extract is not particularly restricted, and methods known per se can be used such as, for example, immersion extraction, heat extraction, continuous extraction, supercritical extraction and the like. The ratio of Ascophyllum to extraction solvent is not particularly restricted, and the ratio of dried Ascophyllum substance/solvent is preferably about 1/100 to 1/2 (w/v), more preferably about 1/10 to 1/5 (w/v). Specifically, extraction is preferably carried out with gentle stirring or allowing to stand using an extraction solvent in an amount of about 200 mL to 10 L, preferably about 500 mL to 1 L based on about 100 g of the extraction raw material which is obtained by, for example, drying and grinding Ascophyllum. It is convenient in view of operability that the extraction temperature is in a range from room temperature to not higher than the boiling point of the solvent under normal pressure, and the extraction time varies depending on the extraction temperature and the like, and is in a range from several minutes to about 7 days, and preferably from about 30 minutes to 24 hours.

After completion of the extraction operation, solid (extraction residue) is removed by methods known per se such as filtration, centrifugation and the like, thereby to obtain an extract. The extract is concentrated by a method known per se, thereby to obtain an extract concentrated in the form of black to brown oil or paste (hereinafter, referred simply to as concentrate in some cases). The extract or concentrate can also be converted into a solid extract by performing drying by methods known per se such as, for example, thermal drying, freeze dry and the like. An extract, concentrate, or solution obtained by dissolving a concentrate in water and/or organic solvent may be purified by a method such as, for example, ultrafiltration, adsorption resin treatment, molecule chromatography, partition chromatography, liquid-liquid extraction and the like. The purified extract can be used for the present invention in the form of purified substance.

The extract according to the present invention is useful as a glycosidase inhibitor since it has a strong $\alpha$-amylase

inhibitory action, and further an α-D-glucosidase inhibitory action.

The above-mentioned glycosidase is classified into two. One is an enzyme hydrolyzing a simple carbohydrate which generates only a sugar by hydrolysis, and the other is an enzyme hydrolyzing a complex carbohydrate which generates also a substance other than sugars. The glycosidase in the present invention means an enzyme hydrolyzing an O-glycosyl compound composed of only sugars. Examples of such a glycosidase include α-amylase, α-D-glucosidase, β-D-glucosidase, sucrase, maltase, isomaltase, lactase, trehalase and the like.

Regarding the glycosidase inhibitor of the present invention, it is preferable that the above-mentioned extract or purified substance is used as it is, or a pharmaceutically acceptable additive, or a food material, food raw material, further if necessary, a food additive and the like are appropriately mixed with the extract or purified substance, and they are preferably formulated into a dosage form such as liquid, powder, granule, tablet, microcapsule, soft capsule, hard capsule and the like by methods known per se. Moreover, it is possible to make into any food and drink forms such as solid food, semisolid food like cream or jam, food like gel, beverage and the like. Examples of such foods and drinks include refreshing beverage, coffee, tea, milk-contained beverage, lactic acid bacteria beverage, drop, candy, chewing gum, chocolate, gummy candy, yoghurt, ice cream, pudding, soft adzuki-bean jelly, jelly, cookie and the like. These various preparations or foods and drinks are useful as a healthy food or food for specified health uses for the treatment and prevention of diabetes.

As the additive, food material, food raw material and food additive used for production of the above-mentioned preparations or foods and drinks, for example, excipients (lactose, corn starch, white sugar, glucose, starch, crystalline cellulose and the like), lubricants (magnesium stearate, sucrose fatty ester and the like), disintegrators (starch, carmellose sodium, calcium carbonate and the like), binders (starch paste liquid, hydroxypropyl cellulose liquid, gum arabic liquid and the like), emulsifiers and solubilizing agents (gum arabic, polysorbate 80, povidone and the like), sweeteners (white sugar, fructose, simple syrup, honey and the like), coloring gents (edible tar pigment, iron oxide and the like), preservatives (methyl p-oxybenzoate, propyl p-oxybenzoate, sorbic acid and the like), thickeners (hydroxyethyl cellulose, polyethylene glycol, sodium alginate and the like), antioxidants (sodium hydrogen sulfite, sodium edetate, ascorbic acid and the like), stabilizers (sodium thiosulfate, sodium edetate, sodium citrate and the like), acidulants (lemon juice and the like), seasonings (sodium glutamate and the like), aromas (mint, strawberry aroma and the like), and the like can be used.

The addition amount of the above-mentioned extract or purified substance based on the above-mentioned various preparations or foods and drinks is not uniform and varies depending on the content of a glycosidase inhibiting component contained in the extract or purified substance, and the amount of the extract (calculated as the solid) is, for example, about 0.0001 to 50% by mass, preferably about 0.001 to 20% by mass, more preferably about 0.01 to 10% by mass.

When these various preparations or foods or drinks are taken orally, the daily dose of the above-mentioned extract or purified substance is about 0.01 to 1000 mg, preferably about 0.1 to 500 mg, further preferably about 1 to 300 mg per kg of body weight when calculated as the solid. This dose may be advantageously taken in one time or several times per day. However, actual dose should be determined in view of the object and conditions of a person who takes it (sex, age, body weight, BMI and the like).

Preferable examples in the present invention are described below, but the scope of the invention is not limited to these examples.

Example 1

About 50.0 g of dried Ascophyllum powder was precisely weighed and to this dried powder was added 500 mL of an ethanol-water mixed solution at ratio shown Table 1, and extraction was performed for 1 hour at room temperature with gentle stirring. The extract was transferred to a centrifuge tube, and divided into a supernatant and a precipitate by centrifugation, and 500 mL of the ethanol-water mixed solution was added to the precipitate, and extraction was performed for 1 hour in the same manner as in the first operation. The extraction solution was divided into a supernatant and a precipitate in the same manner as in the first operation, and the supernatants of the first and second operations were combined and filtered under suction, thereby to obtain an extract in a total volume of about 1 L as a filtrate. This extract was concentrated at about 60°C under reduced pressure using a rotary evaporator, and the concentrate was freeze-dried to obtain extracts 1 to 6 in the form of black brown powder. Each yield is shown in Table 1.

Table 1

| Extract | Ethanol-water mixed solution (ethanol:water (v/v)) | Yield (% by mass) |
|---|---|---|
| Extract 1 | 10:90 | 24.2 |

(continued)

| Extract | Ethanol-water mixed solution (ethanol:water (v/v)) | Yield (% by mass) |
|---|---|---|
| Extract 2 | 20:80 | 24.3 |
| Extract 3 | 30:70 | 24.3 |
| Extract 4 | 50:50 | 22.0 |
| Extract 5 | 70:30 | 17.0 |
| Extract 6 | 100:0 | 2.2 |

Example 2

The $\alpha$-amylase, maltase and sucrase inhibitory activities of the extract obtained in Example 1 were measured.

1) Measurement of $\alpha$-amylase inhibitory activity

1 mL of sample solutions containing the extract obtained in Example 1 in an amount of 5, 10, 15, 20 and 25 ppm respectively through gradual dilution, and 1 mL of 4% by mass of starch solution (dissolved in 0.1 M phosphate buffer (pH 7.0)) were mixed sufficiently, and heated at 37°C for 5 minutes. Then, 0.02 mL (4.25 units/0.02 mL) of an $\alpha$-amylase (Sigma) solution was added and mixed sufficiently, and the mixture was reacted at 37°C for 60 minutes, and kept for 10 minutes in a boiling water bath to stop the reaction, thereby to obtain a reaction solution. In control group 1, an $\alpha$-amylase solution deactivated previously by keeping for 10 minutes in a boiling water bath was added, and in control group 2, water was added instead of a sample solution.

In the sample group and control group 1, the reaction solution was diluted 40-fold, and in the control group 2, the reaction solution was diluted 4-fold, and to 0. 8 mL of the resulting diluted solution was added 0.8 mL of a 0.01 N iodine solution (0.02 N iodine solution (manufactured by Wako Pure Chemical Industries, Ltd.) was diluted 2-fold with distilled water) and 4 mL of distilled water, and the mixture was stirred well to give a test solution. Then, the absorbance of the test solution was measured at a wavelength of 660 nm and at a length of liquid layer of 1 cm using distilled water as a control.

Separately, a calibration curve was made from absorbance values of the liquid which was colored in the same manner as described above using 0.8 mL each of standard solutions containing 50 to 800 $\mu$g of starch in 1 mL. From the abosorbance and calibration curve of the test solution, the remaining starch amount in the reaction solution was obtained, and the inhibition ratio was calculated according to the following equation.

$$\text{Degradation rate (\%)} = (B'-S')/B' \times 100$$

S' : remaining starch amount in reaction solution in sample group ($\mu$g/mL)
B' : remaining starch amount in reaction solution in control group 1 ($\mu$g/mL)

$$\text{Inhibition rate (\%)} = (C-S)/C \times 100$$

S: degradation rate (%) in sample group
C: degradation rate (%) in control group 2

2) Measurement of maltase inhibitory activity

(Preparation of crude enzyme solution)

To a rat intestinal acetone powder (Sigma) was added 18-fold amount (wt) of 0.1 M maleate buffer (pH 6.0), and the mixture was homogenized while cooling with ice and then centrifuged (about 0°C, 3000 rpm, 10 minutes) to give a supernatant, which was then diluted 10-fold (volume) with 0.1 M maleate buffer (pH 6.0) to obtain a crude enzyme solution.

(Measurement of inhibitory activity)

0.2 mL of sample solutions containing the extract obtained in Example 1 in an amount of 0.05, 0.1, 0.2 and 0.4% by mass respectively through gradual dilution, and 0.2 mL of 2% by mass of maltose solution (dissolved in 0.1 M maleate buffer (pH 6.0)) were mixed sufficiently, and heated at 37°C for 5 minutes. Then, 0.2 mL of the crude enzyme solution was added and mixed sufficiently, and reacted at 37°C for 60 minutes, then, kept for 10 minutes in a boiling water bath to stop the reaction. The reaction solution was allowed to cool to room temperature, then, centrifuged (about 20°C, 3000 rpm, 10 minutes) to obtain a supernatant. In control group 1, a crude enzyme solution deactivated previously by keeping for 10 minutes in a boiling water bath was added, and in control group 2, water was added instead of a sample solution.

In the sample group, control group 1 and control group 2, the glucose amount in each supernatant was measured using a measuring kit by a glucose oxidase method (Glucose CI I-Test Wako; manufactured by Wako Pure Chemical Industries, Ltd.), and the inhibition rate was calculated according to the following equation.

$$\text{Inhibition rate (\%)} = (C-(S-B))/C \times 100$$

S: glucose amount in supernatant in sample group (mg/100 mL)
C: glucose amount in supernatant in control group 2 (mg/100 mL)
B: glucose amount in supernatant in control group 1 (mg/100 mL)

3) Measurement of sucrase inhibitory activity

(Preparation of crude enzyme solution)

To a rat intestinal acetone powder (Sigma) was added 18-fold amount of 0.1 M maleate buffer (pH 6.0) and the mixture was homogenized while cooling with ice and then centrifuged (about 0°C, 3000 rpm, 10 minutes) to give a supernatant which was used as a crude enzyme solution.

(Measurement of inhibitory activity)

0.2 mL of sample solutions containing the extract obtained in Example 1 in an amount of 0.05, 0.1, 0.2 and 0.4% by mass respectively through gradual dilution, and 0.2 mL of 2% by mass of sucrose solution (dissolved in 0.1 M maleate buffer (pH 6.0)) were mixed sufficiently, and heated at 37°C for 5 minutes. Then, 0.02 mL of the crude enzyme solution was added and mixed sufficiently and reacted at 37°C for 60 minutes, then, kept for 10 minutes in a boiling water bath to stop the reaction. The reaction solution was allowed to cool to room temperature, then, centrifuged (about 20°C, 3000 rpm, 10 minutes) to give a supernatant. In control group 1, a crude enzyme solution deactivated previously by keeping for 10 minutes in a boiling water bath was added, and in control group 2, water was added instead of a sample solution.

In the sample group, control group 1, and control group 2, the glucose amount in the supernatant was measured using a measuring kit by a glucose oxidase method (Glucose CII-Test Wako; manufactured by Wako Pure Chemical Industries, Ltd.), and the inhibition rate was calculated according to the following equation.

$$\text{Inhibition rate (\%)} = (C-(S-B))/C \times 100$$

S: glucose amount in supernatant in sample group (mg/100 mL)
C: glucose amount in supernatant in control group 2 (mg/100 mL)
B: glucose amount in supernatant in control group 1 (mg/100 mL)

The inhibitory activity is expressed in terms of a concentration ($IC_{50}$) required for 50% inhibition of each enzyme activity. The results of the α-amylase inhibitory activity, maltase inhibitory activity and sucrase inhibitory activity on the extract are shown in Table 2.

Table 2

| Extract | Inhibitory activity (IC$_{50}$) | | |
|---|---|---|---|
| | $\alpha$-amylase | Maltase | Sucrase |
| Extract 3 | 128.5 | 987.5 | 1723.7 |
| Extract 4 | 170.4 | 1119.7 | 1896.5 |
| Extract 5 | 173.8 | 1284.0 | 1765.3 |
| Extract 6 | 42.8 | 723.4 | 923.2 |
| (unit: $\mu$g/mL) | | | |

Table 2 teaches that the extracts 3 to 6 obtained in Example 1 have an $\alpha$-amylase inhibitory activity and an $\alpha$-D-glucosidase inhibitory activity, and particularly, have a strong $\alpha$-amylase inhibitory activity.

Example 3

About 50.0 g of dried powders of various marine algae was precisely weighed, and to these dried powders were added 500 mL each of an ethanol-water (30:70 (v/v)) mixed solution, and extraction was performed for 1 hour at room temperature with gentle stirring. The extract was moved to a centrifuge tube, and divided into a supernatant and a precipitate by centrifugation, and 500 mL of the ethanol-water (30:70 (v/v)) mixed solution was added to the precipitate, and extraction was performed for 1 hour in the same manner as in the first operation. The extract was divided into a supernatant and a precipitate in the same manner as in the first operation, and the supernatants of the first and second operations were combined and filtered under suction, giving an extract in a total volume of about 1 L as a filtrate. This extract was concentrated at about 60°C under reduced pressure using a rotary evaporator, and the concentrate was freeze-dried to obtain extracts (extract 7, Comparative Examples 1 to 8) in the form of powder.
The $\alpha$-amylase inhibitory activity of these extracts was measured according to Example 2 described above. The concentration of the sample solution was 10 $\mu$g/mL, 100 $\mu$g/mL or 1000 $\mu$g/mL. The results are shown as inhibition rate (%) in Table 3.

Table 3

| | Kind of marine algae | Inhibition rate (%) | | |
|---|---|---|---|---|
| | | 10 $\mu$g/mL | 100 $\mu$g/mL | 1000 $\mu$g/mL |
| Extract 7 | Ascophyllum nodsum (brown algae) | 25.4 | 94.4 | 95.3 |
| Comparative Example 1 | Ulva pertusa Kjellman (green algae) | 0 | 0 | 0 |
| Comparative Example 2 | Nemacystus decipiens (brown algae) | 0 | 0 | 0 |
| Comparative Example 3 | Laminaria japonica (brown algae) | 0 | 8.2 | 95.8 |
| Comparative Example 4 | Eisenia bicyclis (Kjellman) Setchell (brown algae) | 0 | 0 | 0 |
| Comparative Example 5 Pinnatifida | Undraia (brown algae) | 0 | 0 | 8.9 |
| Comparative Example 6 | Sargassum fulvellum (brown algae) | 0 | 0 | 4.6 |
| Comparative Example 7 | Hizikia fusiformis (brown algae) | 0 | 0 | 0 |
| Comparative Example 8 | Ptilophora subcostata (red algae) | 0 | 0 | 0 |

From Table 3, it can be seen that an extract of Ascophyllum has a stronger $\alpha$-amylase inhibitory activity as compared with other marine algae, and additionally, its activity is manifested even at lower concentrations.

Example 4

Rat glucose tolerance test

A glucose tolerance test in rats was performed using, as a sample, the extract 1, extract 3 and extract 4 obtained in Example 1. Each five 9-week old Wistar rats fasted overnight were used in a control group and a sample administration group. About 0.5 mL of blood was collected from the rat tail vein into a heparin-containing blood collection tube. After blood collection, starch and samples so mixed and prepared as to give starch 1 g/body weight kg in the control group and starch 1 g/body weight kg and sample 1 g/body weight kg in the sample administration group were orally administered each using a gastric sonde. About 0.5 mL of blood was collected 30, 60 and 120 minutes after the administration. The collected blood was centrifuged to give a plasma fraction, and was preserved at -40°C until analysis.

The plasma glucose level was measured by a glucose oxidase method using a measuring kit (Glucose CII - Test Wako; manufactured by Wako Pure Chemical Industries Ltd.). The change with time of the plasma glucose level is shown in Table 4.

Table 4

| Elapsed time (minute) | Plasma glucose level (mg/100 mL) (numerical value is average value $\pm$ standard deviation) | | | |
| --- | --- | --- | --- | --- |
| | Control group | Administration group (1) | Administration group (2) | Administration group (3) |
| 0 | 117 $\pm$ 17 | 120 $\pm$ 9 | 119 $\pm$ 17 | 118 $\pm$ 9 |
| 30 | 166 $\pm$ 11 | 138 $\pm$ 14 * | 138 $\pm$ 14 * | 144 $\pm$ 7 * |
| 60 | 154 $\pm$ 8 | 130 $\pm$ 10 * | 136 $\pm$ 26 | 145 $\pm$ 13 |
| 120 | 121 $\pm$ 15 | 121 $\pm$ 2 | 127 $\pm$ 12 | 121 $\pm$ 6 |
| * significant difference with a crisis ratio of 1% for the control group administration group (1): extract 1 in Example 1 is administered as sample administration group (2): extract 3 in Example 1 is administered as sample administration group (3): extract 4 in Example 1 is administered as sample | | | | |

It was found that the extract of the present invention suppresses significantly increase in the plasma glucose level 30 minutes after administration of starch as compared with the control group.

Example 5

To about 800 g of Ascophyllum dried powder was added 8 L of an ethanol-water (50:50 (v/v)) mixed solution, and extraction was performed for 1 hour at room temperature with gentle stirring. The extract was moved to a centrifuge tube, and divided into a supernatant and a precipitate by centrifugation, and 8 L of the ethanol-water mixed solution was added to the precipitate, and extraction was performed for 1 hour in the same manner as in the first operation. The extract was divided into a supernatant and a precipitate in the same manner as in the first operation, and the supernatants of the first and second operations were combined and filtered under suction to obtain an extract in a total volume of about 16 L as a filtrate. This extract was ultrafiltered using an ultrafiltration membrane having a fractional molecular weight of 10000 (trade name: FB02-VC-FUSO181; Daicen Membrane Systems), and when the amount of the concentrated solution reached a volume of 5 L, 5 L of water was added and filtration was continued, and when the amount of the concentrated solution reached again 5 L, ultrafiltration was stopped. The concentrated solution was concentrated at about 60°C under reduced pressure using a rotary evaporator, and the concentrate was freeze-dried to obtain about 73 g of an extract (extract 8) in the form of black brown powder.

Example 6

To 50 parts by mass of lactose, 38 parts by mass of corn starch, 1 part by mass of lemon aroma and 1 part by mass of sucrose fatty acid ester were added 10 parts by mass of the extract 8 in Example 5, and they were mixed, and then the mixture was tabletted using a tabletting machine to produce a supplement.

Example 7

A beverage solution having composition shown in Table 5 was heated at about 65°C for 10 minutes, and after being cooled down to room temperature, the solution was filled aseptically in a sterile container to produce an apple juice beverage.

Table 5

| Component | Addition amount (mass%) |
|---|---|
| Fructose-glucose syrup | 14 |
| Apple transparent juice | 10 |
| Aroma | 0.2 |
| Acidulant | 0.15 |
| Vitamin C | 0.03 |
| Pigment | 0.01 |
| Extract 8 of Example 5 | 1.00 |
| Water | 74.61 |
| Total | 100 |

Example 8

Coffee jelly was produced according to the following procedure.

(1) 15 g of gelatin powder is placed in about 45 mL of water and swollen.
(2) 600 mL of water, 3 g of instant Coffee and 80 g of granulated sugar are put in a pan and boiled. When the granulated sugar is dissolved, fire is extinguished, and about 7 g of the extract 8 of Example 5 and the above (1) are added and the mixture is dissolved well.
(3) The product is cooled and about 10 mL of brandy is added thereto, and the mixture is cooled until thickened, then, poured into a jelly mold of which inside wall has been moistened, and cooled to solidify.

INDUSTRIAL APPLICABILITY

An extract from Ascophyllum nodsum obtained in the present invention has a strong glycosidase inhibitory action. Therefore, a glycosidase inhibitor containing the extract from Ascophyllum nodsum can obtain a more effective effect on the treatment and/or prevention of diebetes as compared with conventionally known glycosidase inhibiting substances derived from marine algae. Moreover, a food and drink containing the above-mentioned extract is useful as a healthy food or food for specified health uses for the treatment and/or prevention of diabetes.

**Claims**

1. A glycosidase inhibitor comprising an extract of Ascophyllum nodsum as an active ingredient.

2. A glycosidase inhibitor comprising a purified substance of the extract according to claim 1 as an active ingredient.

3. The glycosidase inhibitor according to claim 1 or 2, which is in the form of food and drink.

4. The glycosidase inhibitor according to claim 3, which is in the form of healthy food or food for specified health uses for the treatment and/or prevention of diabetes.

5. A method of inhibiting glycosidase, which comprises administering an extract of Ascophyllum nodsum to a mammal.

6. A method of treating or preventing diabetes, which comprises administering an extract of Ascophyllum nodsum to a mammal.

7. Use of an extract of Ascophyllum nodsum for producing a medicine or food and drink which inhibits glycosidase.

8. Use of an extract of Ascophyllum nodsum for producing a medicine or food and drink for treating or preventing diabetes.

## INTERNATIONAL SEARCH REPORT

| International application No. |
|---|
| PCT/JP2004/018370 |

**A. CLASSIFICATION OF SUBJECT MATTER**
Int.Cl⁷ A61K35/80, A23L1/30, A61P3/10, 43/00, C12N9/99

According to International Patent Classification (IPC) or to both national classification and IPC

**B. FIELDS SEARCHED**

Minimum documentation searched (classification system followed by classification symbols)
Int.Cl⁷ A61K35/80, A23L1/30, A61P3/10, 43/00, C12N9/99

Documentation searched other than minimum documentation to the extent that such documents are included in the fields searched

Electronic data base consulted during the international search (name of data base and, where practicable, search terms used)
CA(STN), BIOSIS(STN), MEDLINE(STN), EEMBASE(STN), JICST(JOIS)

**C. DOCUMENTS CONSIDERED TO BE RELEVANT**

| Category* | Citation of document, with indication, where appropriate, of the relevant passages | Relevant to claim No. |
|---|---|---|
| A | WO 2002/22140 A1 (Takara Bio Inc.), 21 March, 2002 (21.03.02), Claim 5; page 6, line 27 & AU 880400 A & EP 1327448 A1 & US 2004/29828 A1 | 1-4,7,8 |
| A | JP 5-284937 A (Kabushiki Kaisha TAC Gijutsu Kagaku Kenkyusho), 01 November, 1993 (01.11.93), Table 1 (Family: none) | 1-4,7,8 |
| A | JP 2002-212095 A (Hokkaido), 31 July, 2002 (31.07.02), (Family: none) | 1-4,7,8 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search 27 December, 2004 (27.12.04) | Date of mailing of the international search report 18 January, 2005 (18.01.05) |
|---|---|
| Name and mailing address of the ISA/ Japanese Patent Office | Authorized officer |
| Facsimile No. | Telephone No. |

Form PCT/ISA/210 (second sheet) (January 2004)

**INTERNATIONAL SEARCH REPORT**

| | International application No. |
|---|---|
| | PCT/JP2004/018370 |

C (Continuation). DOCUMENTS CONSIDERED TO BE RELEVANT

| Category* | Citation of document, with indication, where appropriate, of the relevant passages | Relevant to claim No. |
|---|---|---|
| A | Hideyuki KIRIHARA et al., A News Screening Method for Glucosidase Inhibitors and Application to Algae Extracts, Fisher Science, 1994, Vol.60, No.6, pages 759 to 761, tables 2, 3 | 1-4,7,8 |
| A | Ermakova, S.P. et al., Proteins of brown seaweeds as inhibitors of endo-1→3-β-D-glucanases of marine invertebrates, Biochemistry(Moscow,Russian Federation), 2001, Vol.66, No.2, pages 188 to 194, abstract. [On line]:STN).Chemical Abstracts, AN.135:251385 | 1-4,7,8 |

Form PCT/ISA/210 (continuation of second sheet) (January 2004)

## INTERNATIONAL SEARCH REPORT

International application No.

PCT/JP2004/018370

---

**Box No. II        Observations where certain claims were found unsearchable (Continuation of item 2 of first sheet)**

This international search report has not been established in respect of certain claims under Article 17(2)(a) for the following reasons:

1. ☒ Claims Nos.: 5, 6
   because they relate to subject matter not required to be searched by this Authority, namely:
   The inventions as set forth in claims 5 and 6 pertain to methods for treatment of the human body by therapy.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III        Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**        ☐ The additional search fees were accompanied by the applicant's protest.

☐ No protest accompanied the payment of additional search fees.

---

Form PCT/ISA/210 (continuation of first sheet (2)) (January 2004)

13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5284937 A
- JP 2000342224 A
- JP 2002212095 A
- JP 57140727 A
- JP 7059539 A
- JP 12072682 A
- JP 2000063281 A
- JP 9291039 A